Europäisches Patentamt

European Patent Office

Office européen des brevets

(19)

(11) Publication number: **0 115 459**
**A2**

# (12) EUROPEAN PATENT APPLICATION

(21) Application number: **84400146.1**

(22) Date of filing: **24.01.84**

(51) Int. Cl.³: **G 01 N 33/68**

(30) Priority: **26.01.83 US 440540**
**04.10.83 US 538783**

(43) Date of publication of application:
**08.08.84 Bulletin 84/32**

(84) Designated Contracting States:
**AT BE CH DE FR GB IT LI LU NL SE**

(71) Applicant: **UNIVERSITY OF MEDICINE AND DENTISTRY OF NEW JERSEY**
**100 Bergen Street**
**Newark New Jersey(US)**

(72) Inventor: **Spillert, Charles R.**
**10 Edgemont Rd.**
**West Orange New Jersey(US)**

(72) Inventor: **Suval, William D.**
**209 Clinton Pl.**
**Hackensack New Jersey(US)**

(72) Inventor: **Lazaro, Eric J.**
**128 Kensington Ave.**
**New Jersey(US)**

(74) Representative: **Descourtieux, Philippe et al,**
**CABINET BEAU de LOMENIE 55 rue d'Amsterdam**
**F-75008 Paris(FR)**

(54) **Hematological prediction of sepsis and other disease states.**

(57) A new diagnostic procedure employing a prepacked assay kit to enable the hematological prediction of cancer, sepsis, AIDS and other pathologic states is described. The kits are composed of sterile plastic test tubes and caps wherein a control tube contains an appropriate amount of physiologic saline, and an activated tube contains various activators of specific pathways of blood coagulation, e.g. endotoxins dissolved in the same volume of saline. Upon the addition of a patient's citrated whole blood (CWB) to each tube, incubation and measurement of recalcification times (RT) are performed. A ratio of the control sample RT divided by the activated samples (endotoxin) RT yields a thrombotic index (TI), the values of which are related to various disease states. Low numerical values of this index (if endotoxin is the activating material at 10μg/cc CWB) indicate post surgical patients at high risk for sepsis (TIμ1.60); values above 1.60 indicate post-surgical patients at low risk for sepsis. Normal volunteers have values ranging from 1.15 - 1.53 whereas newly diagnosed cancer patients have values of 1.62 to 4.39 or higher.

# HEMATOLOGICAL PREDICTION OF SEPSIS AND OTHER DISEASE STATES

## BACKGROUND OF THE INVENTION

Sepsis (bacterial infections), in spite of the introduction of new generations of antibiotics, remains the most serious problem to patients assigned to surgical and medical intensive care units. Many surveys disclose that more than 50% of the patients who die in these units succumb to sepsis and/or resultant multisystem organ failure. There is currently no dependable, rapid, single test that indicates which patients will develop septic complications, or that predicts survival or efficacy of therapy in early endotoxic states.

Cancer detection by means of a simple, reliable, specific blood test heretofore has not been known. The potentional value of a diagnostic cancer detection test would lead to improved prognosis, less radical treatment to cure some early cases, reduced suffering and reduced costs for health care.

Researchers have established that when human monocytes are treated with immunomudulators such as endotoxin, procoagulant activity is generated. Furthermore, the monocytes isolated from many cancer patients produce more of this procoagulant activity than do the monocytes from individuals with no malignancy.

However, these tests are complicated, require elaborate equipment, lengthy technician time, employ artificial growth media and are not adaptable to the clinical situation. In addition, the numerical value of an individual's procoagulant activity alone, as measured by these tests, has little diagnostic significance.

## SUMMARY OF THE INVENTION

In general, the invention is concerned with a diagnostic method of predicting the presence of cancer, sepsis, AIDS and other pathological states. The method includes the steps of transferring a portion of anticoagulated blood to a first container having therein a suitable vehicle for serving as a control, transferring another portion of said anticoagulated blood to a second container having therein an activating agent of the blood coagulation system in a suitable vehicle, and then incubating the mixtures of the first and second containers at a predetermined temperature for a relatively short period of time. A clotting parameter for said mixtures is then procured so as to establish a thrombotic index or percentage of clotting time.
Finally, the resultant relationship of said thrombotic index or percentage of clotting time is evaluated to predict the presence of cancer, sepsis, AIDS or other pathological states.

The invention also is concerned with providing a prepackaged diagnostic array or kit for predicting the presence of cancer, sepsis, AIDS and other pathological states. The kit includes a first container having a predetermined vehicle therein for receiving a portion of anticoagulated blood, said resultant mixture to serve as a control. A second container is provided with an activating agent of the blood coagulation system for receiving another portion of said anticoagulated blood therein. The mixture of the first and second containers are then subjected to a predetermined temperature for a relatively short period of time to procure a clotting parameter for each of said mixtures for establishing a thrombotic index or percentage of clotting time. Means are provided for evaluating the resultant relationship of said thrombotic index or percentage of clotting time to predict the presence of cancer, sepsis, AIDS or other pathological states.

Specifically, we describe a diagnostic procedure wherein citrated whole blood is employed, only minutes of technician time are required and results can be obtained within 2.0 hours after the sample is drawn. Furthermore, the results enable the accurate prediction of the presence of the great majority of cancers and aids in the diagnosis of other pathologic states including sepsis (pus).

Aliquots of blood are incubated with either saline (control) or endotoxin (activator) for two hours. The endotoxin stimulates production of a greater amount of procoagulant activity than that of the control tube with no activator. The effect of this procoagulant activity on clotting can then be quantitated by measuring the shortening of the recalcification time (RT) of the activated sample (RTe) compared to that of the controls (RTs). In addition, most cancer patients have markedly reduced RTe values than do either normal individuals or patients without cancer, while the values of RTs are all similar. Therefore, the thrombotic index (TI), RTs $\div$ RTe, is greater for cancer patients, as is the percentage shortening of clotting (PSOC) of the RT induced by endotoxin, PSOC=(RTs$\div$RTe) 100 $\div$ RTs.

In normal individuals no significant changes in TI or PSOC were observed for variations in age, sex, smoking habits or drugs consumed other than anticoagulants.

The cancers detected include lung, breast, biliary, bladder, laryngeal, ovary, head and neck, colon, rectal, esophageal, soft palate, pancreas, floor of mouth and gallbladder. In addition, the presence or absence of remaining malignancy after curative surgery can be evaluated by this procedure.

Early stages of cancer have profound effects on activating the immune system. Since this diagnostic test quantitates some of the major aspects of the immune system it is expected that other diseases which affect the immune system can also be evaluated. It has been found that the immunosuppressive disease states, such as AIDS (Acqui-
red Immune Deficiency Syndrome) and sepsis (pus), can
be diagnosed in part by this test method.

In addition, by the use of procoagulants, such as
thromboplastin, thrombin or viper venom as the activators,
states of accelerated coagulation (prethrombotic or hypercoagulable blood states) can be evaluated by this test.

A method has been discovered by which a thrombotic
index is determined for the individual patient dependent
only upon the characteristics of his blood, the activator
of the specific pathway of blood coagulation chosen, e.g.
endotoxin, and the concentration of this activator and
the time of incubation.

Depending upon the activator chosen, e.g. endotoxin,
the value of the thrombotic index (TI) predicts whether
the patient either has cancer, AIDS or is at thrombotic
risk, e.g. coronary artery disease or diabetes. Also,
the TI value predicts whether a post-operative patient
may become septic. Furthermore, these tests give repro-
ducible results and is independent of the patient's age,
sex, drugs administered, cardiac and respiratory irregularities, kidney failure, and other associated variables.

Lower numerical values of this index (if endotoxin
is the activating material at $10\mu g/cc$ CWB) indicate
(1.15-1.53) normal values ; higher values (1.62 to 4.39
or higher) are indicative of cancer. It may be desirable
to use percent shortening of clotting (PSOC) time induced
by activator rather than the index just described. Thus,
(control RT-activated RT/control RT) x 100 would yield
the following : 14.0 - 35.0 % normal values whereas,
cancer patient values are 38.3 - 88.0 % or higher. The
normal values and values for cancer patients do not overlap. The use of the control tube increases the sensitivity of the method and helps diagnose coagulopathies.

The same test has been employed detecting septic (pus present) patients from non-septic patients post surgery or trauma. Using a separation value of TI=1.30 equivalent to PSOC=23.0%, we find 8 of 10 patients with values below 1.30 with confirmed pus, whereas only 2 of 62 patients with values above 1.30 were found to have pus.

Since these tests are indirectly measuring immunocompetence, it was found that if a patient survived surgery or trauma, the mean TI was 2.75, whereas if death occured, the TI was only 1.49. Those that died succumbed to multisystem failure which resulted from sepsis.

Acquired Immune Deficiency Syndrome (AIDS) patients are severely immunocompromised. Three of four confirmed AIDS patients had PSOC values below the lowest value of normal (14.0) individuals.

In addition, blood or plasma samples with accelerated coagulation (hypercoagulable) have been evaluated using this technique but also employing threshold quantities of procoagulants such as thromboplastin or Russel's viper venom as activators.

Since this test measures immunocompetence, the following potential uses may be considered :

a. screen for bloods donated to blood banks for human use ;

b. monitor for early signs of transplanted organ rejection ;

c. monitor the effect of therapies on disease state ; and,

d. monitor pregnancies for possibilities of rejection.

e. in Vitro assessment of potential therapies against disease states.

Since this test procedure is very sensitive and selective to many abnormalties associated with the clotting of bloods, pre-packaged arrays or kits are described incorporating these relevant features needed to facilitate the widespread use of this diagnostic test.

## DETAILED DESCRIPTION OF THE INVENTION
## DETERMINATION OF THROMBOTIC INDEX (TI)
## OR PERCENT SHORTENING OF CLOTTING (PSOC)

Plasticware is used throughout the procedure.

The patient's blood is collected via a clean venipuncture using a syringe and 20 gauge needle without stasis or the undue use of force to draw blood into the syringe. The blood is immediately but gently transferred to a tube containing 3.8% sodium citrate (9 parts blood: 1 part citrate) and inverted gently several times to mix. Two cc of this citrated whole blood (CWB) is then transferred to plastic tubes (12 x 75 mm with caps) as follows:

Tube A, containing $20\mu l$ of a suitable vehicle, such as physiological saline (control tube);

Tube B, containing $20\mu l$ of a 1 mg/cc suspension of E. Coli endotoxin (strain O55:B5) in a suitable vehicle, such as saline.

Additional tubes containing other concentrations of activating agents may be employed, as well as additional activators. These tubes are gently mixed, capped and incubated at 37 degrees C for approximately two hours. The citrated whole bloods are then subjected to standard "tilt tube" recalcification time (RT) or preferably to viscoelastic measurements of clot formation, such as those utilized by the thromboelastograph (TEG) or Sonoclot. These instruments are very sensitive to fibrin formation and will markedly improve the sensitivity and reproducibility of this test. If a TEG is employed, 0.5cc CWB is mixed with 0.1cc of 0.035M $CaCl_2$ in a plastic tube and 0.36cc of the mixture added to the cuvette, and the "R" values determined, as per instrument instructions ; that is, the time necessary for first fibrin strands to form (minutes). The TI = R (saline)/R (endotoxin). If a Sonoclot is employed, $10\mu\ell$ of 0.5M $CaCl_2$ is mixed with 0.4cc of the CWB. The time necessary to yield a threshold value of 10% full scale deflection is taken as the end-point. This 10% value corresponds directly with the TEG "R" values. The percent shortening of clotting time (PSOC) induced by endotoxin ;

$$PSOC = R\frac{\text{(saline)} - R \text{ (endotoxin)} \times 100}{R \text{ (saline)}}$$ is significantly elevated in cancer patients, reduced in AIDS patients elevated 24-48 hours post surgery or trauma in patients that survive, and not elevated in trauma and surgery patients that die.

If other activators are employed, thrombin, thromboplastin or viper venom, then individuals of increased thrombotic risk will have lower TI or PSOC than individuals with less risk.

Although the tests employed were RT of citrated (oxalate was not as good as citrate) whole blood, it may be possible after the incubation period of the samples to perform other clotting tests or centrifuge, and perform coagulation tests on the resulting platelet poor plasma, or cellular elements, or to add these items to another test system and get clinically relevant data. However, any other system will still directly or indirectly measure activated coagulation (or inhibited coagulation) and would be covered by the art of this application.

In non-septic bloods, the incubation with endotoxin should produce a shortened RT on the TEG, since endotoxin produces a procoagulant. However, septic patients' bloods have been exposed to endotoxin in vivo, and thus are not as activated and produce a longer RT than that of non-septic patients.

CANCER DETECTION

In the table below are listed the mean values for Rs, Re, TI and PSOC, as well as the range for 23 cancer patients and 25 normal volunteers.

### MEAN VALUES AND RANGE

| Group | Rs, | range | Re, | range | TI | range | PSOC% | range |
|-------|-----|-------|-----|-------|-----|-------|-------|-------|
| Normal | 6.13 | 5.24-7.61 | 4.66 | 3.93-6.04 | 1.32 | 1.15-1.53 | 23.4 | 14.0-34.5 |
| Cancer | 6.46 | 4.54-8.72 | 2.52 | 1.54-4.02 | 2.71 | 1.62-4.39 | 60.3 | 38.3-88.0 |

The cancer patients were evaluated at the time of diagnosis of the disease or shortly thereafter.

Normal volunteers were of both sexes, age 21-69 years, smokers and non-smokers. No questions were asked as to type or amount of drugs consumed or if they were currently under treatment for any disease.

There were no significant differences between the cancer and volunteer group for the Rs values, whereas the Re values were significantly lower in the cancer group. Whether one employs the TI or the PSOC data, it can be seen that there is no overlap between groups. The maximum TI and PSOC values are 1.53 and 34.5% respectively for the normal group whereas the minimum values for cancer patients is 1.62 and 38.8% respectively.

### BREAST CANCER

| Patient | Rs | Re | TI | PSOC(%) |
|---------|------|------|------|---------|
| 1 | 7.31 | 2.00 | 3.66 | 72.6 |
| 2 | 7.04 | 3.28 | 2.15 | 53.4 |
| 3 | 6.61 | 2.51 | 2.63 | 60.2 |
| 4 | 6.06 | 3.52 | 1.72 | 42.0 |

Besign Breast Lesions

| Patient | Rs | Re | TI | PSOC (%) |
|---------|------|------|------|------|
| 1 | 4.54 | 4.32 | 1.05 | 4.84 |
| 2 | 5.98 | 4.87 | 1.23 | 18.6 |

The above data clearly shows that women with breast cancer have a higher TI or PSOC than those with benign breast disease. In addition, one week after surgery, patients #3 and #4 had values of TI equal to 1.25 and 1.39 respectively and PSOC values of 20.0% and 27.9% respectively. This reduction of the activation of endotoxin-induced clotting was due to successful removal of all malignancy as indicated by a negative spread of cancer to lymph nodes as determined by histological examination of tissue. In cancer cases where a large amount of tumor load is removed but minute malignancy remains, the values of TI or PSOC remain elevated in the cancer range. An example of this would be a patient with colon cancer with values of TI and PSOC of 2.04 and 50.9% respectively, prior to surgery, and values of 1.91 and 47.6 % one week post surgery. Histological evidence indicated that the tumor had invaded nearby tissue.

This test may also enable to clinician to evaluate the effect of therapy on the state of the disease. For example, reduction in TI or PSOC after curative surgery has been demonstrated. However, if chemotherapy and/or radiation treatments do not reduce these parameters, changes in the treatment may be instituted to find a better drug regime and/or radiation protocol. The advantage of this method is that results can be obtained at varying times after therapy and changes observed prior to the appearance of clinical changes.

SEPTIC AND IMMUNOCOMPROMISED PATIENTS (e.g. AIDS)

Despite the availability of several new antibiotic agents and despite advances in diagnostic and therapeutic techniques, the morbidity and mortality resulting from serious infections continues to be high. In the surgical intensive care unit ; patients who have had severe trauma and/or major surgery and who are being managed by life-support systems frequently succumb to multisystem failure resulting from progressive and, perhaps, undetected sepsis. Figures indicate that a significant number of the deaths in this setting are due to overwhelming and uncontrollable bacterial infection. Under these circumstances, once an abscess has formed inside body cavities (abdomen or thorax), antibiotics serve no useful purpose until the pus has been drained because the antibiotics cannot penetrate the abscess wall and/or because the organisms are resistant to the drug. New techniques for identifying abscesses, by computed tomography (CAT scans) or ultrasound, have certain inherent disadvantages : (1) The patient has to be transported to the diagnostic unit for the test. This is hazardous if the patient is critically ill. (2) The cost of conducting the study and the manpower involved place a heavy burden on the already exhausted cost of health care delivery. (3) These tests do not identify that group of patients with infections who are potential candidates for pus formation.

This diagnostic test has been utilized to determine the presence of intra-abdominal abscesses in surgical or trauma patients.

In this group, the TI and PSOC is expected to be elevated compared to that of the normal population due to the thromboplastins released and immunological consequences from the presence of traumatized tissue, (and accordingly bears no relationship to the TI values attained from non-surgery or presurgery patients), as

hereinbelow indicated :

| CATEGORY | THROMBOTIC INDEX | | PSOC | |
|---|---|---|---|---|
| | $< 1.30$ | $> 1.30$ | $< 2.30$ | $> 2.30$ |
| Septic (n=10) | 8 | 2 | 8 | 2 |
| Non-septic (n=62) | 2 | 60 | 2 | 60 |

However, in 80 percent of septic patients (pus), these indices are not elevated as expected. Values of TI lower than 1.30 or PSOC values below 23.0% usually show the presence of pus in the abdomen. This is probably due to the fact that in these individuals, the blood has already been exposed to high endotoxin concentrations (bacterial poisons).

IMMUNOCOMPETENCE.

This diagnostic test has also been utilized to assess immunocompetence in post-operative and post-traumatic patients. Using E. Coli endotoxin as the activator, the blood of 55 patients were evaluated and are tabulated below.

| | MEAN VALUES | | | | |
|---|---|---|---|---|---|
| Groups | n | Rs | Re | TI | PSOC(%) |
| Non-survivors | (9) | 6.72 | 4.51 | 1.49 | 32.9 |
| Survivors | (46) | 6.09 | 2.21 | 2.75 | 63.7 |

There were no significant differences in the Rs values, but highly signifcant differences in the Re, TI, and PSOC values between survivors and non-survivors. Unlike the elevated values of TI and PSOC in cancer patients, elevated values occurring within 48 hours post-operatively or post-trauma are good prognostic indicators. If the values are low post-operatively, one should be aware of possible septic complications.

Furthermore, a TI value of 1.60 signifies a boundary defining a threshold value of immunocompetence, since 8 of 9 patients who died had values below 1.60, whereas only one patient with a value above 1.60 died.

This test measures the immunocompetence of an individual if endotoxin or other possible immunomodulators are employed as activators of the coagulation system. Therefore, activation (accelerated coagulation) in response to endotoxin, of the immune system of cancer patients (early in the disease), and post-operative or post-trauma patients, may in part explain the thrombotic complications associated with these states.

ACQUIRED IMMUNE DEFICIENCY SYNDROME (AIDS).

It is well known that individuals with AIDS or AIDS-like disease states have reduced immunocompetence. These individuals succumb to opportunistic organisms or to rare forms of cancer.
The immunological testing for AIDS involves the isolation of different leukocytes and their subsequent testing. These tests are not only costly and lengthy but are inconclusive as to the presence of AIDS. The test system described herein measures the effects of the interaction of leukocytes with various immunomodulators on the clotting system. Since septic patients are immunocomprised and detected by this test, AIDS patients would be expected to be diagnosed if the appropriate parameters including appropriate activator , activator concentration, and time of incubation were present. Indeed, this has been observed ; AIDS patients have a lower TI and PSOC than normal individuals in 3 of 4 cases.

If this test can be optimized, blood donors or other individuals could be screened for AIDS or for a measure of immune function.

The chart below shows the data obtained from four confirmed AIDS patients and compares that with normal volunteers.

MEAN VALUES AND RANGE

| Group | n | Rs, | Range | Re, | Range | TI, | Range | PSOC, | Range |
|-------|-----|------|-------|------|-------|------|-------|-------|-------|
| Normal | (25) | 6.13 | 5.24-7.61 | 4.66 | 3.93-6.04 | 1.32 | 1.15-1.53 | 23.4 | 14.0-34.5 |
| AIDS | (4) | 5.78 | 5.12-6.94 | 4.94 | 4.43-5.40 | 1.17 | 1.08-1.29 | 14.5 | 7.5-22.2 |

From this data is can be seen that the TI as well as the PSOC for AIDS patients is below that of normal individuals. If PSOC is considered, three of the four AIDS patients have values below that of the lowest normal value. Therefore, if this test is utilized in testing for immunocompetence, the majority of AIDS or AIDS-like diseases will give PSOC values below that of normal individuals and may help in the diagnosis. Furthermore, for individuals donating blood for human use, it may be desirable to have values above some baseline value before the blood is utilized.

DESCRIPTION OF PROPOSED PRE-PACKAGED KITS TO ENABLE DETERMINATION OF THROMBOTIC INDEX (TI)

The previous section details the use of E. Coli endotoxin as an example of a specific activator of blood coagulation when incubated in vitro with citrated whole blood. Materials which also may be employed as activators, and with which kits may be assembled include the appropriate concentrations of different strains (mixtures) of endotoxins (Gram positive or Gram negative) collagens, platelet activating factor (PAF), carrageenans, thromboplastins, and other procoagulants, antigens or other activators of clotting or immunomodulators. Each of these materials may have a different mechanism for activating blood clotting and, therefore, perhaps other clinically significant data will ba attainable.

The kits may contain various size test tubes (e.g. 12 x 75mm for adults to 6 x 50mm for neonates) containing varying concentrations of the different activators.

Also included would be a capped plastic tube with the appropriate amount of citrate to function as the anticoagulant in which to place the blood sample to produce CWB. By having varying concentrations of activators available, the clinical significance of the data may be maximized. Furthermore, the techniques between different laboratories will vary and with a multitude of concentrations available, the best test system for that particular institution can be utilized.

A kit for small office use may contain a tube containing citrate, another saline (control) and another mixed endotoxins in saline, and a third of collagen in saline. There are many possible combinations of reagents to be included depending upon the data needed, but in the kits, one tube must contain only the vehicle so that a ratio or percent shortening of the clotting time can always be attained.

A hospital or large institution may desire a ready to use disposable rack containing a large number of the sealed tubes of one particular reagent or saline in the appropriate size and concentration. The sterile tubes are color coded as to contents with labels affixed.

For large institutions, it may be possible to design an automated device to perform these tests, calculations, and predict the diagnosis.

Under some remote circumstances a kit may contain the tubes previously described, instructions, syringe, needles, pipettes, prep sponges and band-aids. In addition, some tubes may contain heparin to give rise to prolonged RT or a thromboplastin to give shortened RT, so as to verify an abnormal value for each patient tested.

## C L A I M S

1.    A diagnostic method of predicting the presence of cancer, sepsis, AIDS and other pathological states, which includes :

transferring a portion of anticoagulated blood to a first container having therein a suitable vehicle for serving as a control,

transferring another portion of said anticoagulated blood to a second container having therein an activating agent of the blood coagulation system in a suitable vehicle,

incubating the mixtures of the first and second containers at a predetermined temperature for a relatively short period of time,

procuring a clotting parameter for said mixtures so as to establish a thrombotic index or percentage of clotting time, and

evaluating the resultant relationship of said thrombotic index or percentage of clotting time to predict the presence of cancer, sepsis, AIDS or other pathological states.

2.    A diagnostic method, according to Claim 1, wherein the relationship between the resulting clotting parameter is recalcification time.

3.    A diagnostic method, according to Claim 2, wherein a thrombotic index is obtained by dividing the value of the control sample in the first container by the value of the activated sample in the second container.

4.    A diagnostic method, according to Claim 2, wherein a percentage shortening of clotting is obtained by

$$PSOC = \frac{R(s) - R(e) \times 100}{Rs}$$ wherein (s) is the value of the control sample in the first container and (e) is the value of the activated sample in the second container.

5.    A diagnostic method, according to Claim 2, wherein the activating agent of the blood coagulation system is selected from the class consisting of endotoxins, collagens, platelet activating factors, carrageenans, thromboplastins, antigens and other activators of clotting or immunomodulators.

6. .  A diagnostic method, according to Claim 2, wherein the incubating temperature is approximately 37°C.

7.    A diagnostic method, according to Claim 6, wherein the incubating time is approximately 2.0 hours.

8.    A diagnostic method, according to Claim 6, wherein the vehicle in the first container is a saline solution.

9.    A diagnostic method, according to Claim 6, wherein the activating agent in the second container is an endotoxin.

10.    A prepackaged diagnostic array for predicting the presence of cancer, sepsis, AIDS and other pathological states which includes :

a first container having a predetermined vehicle therein for receiving a portion of anticoagulated blood therein to serve as a control,

a second container having an activating agent of the blood coagulation system therein for receiving another portion of said anticoagulated blood therein, said mixtures of the first and second containers being incubated at a predetermined temperature for a relatively short period of time to procure clotting parameters therefor,

and means for establishing a thrombotic index or percentage of clotting time which predicts the presence of cancer, sepsis, AIDS or other pathological states.

11.    A prepackaged diagnostic array, according to Claim 10, wherein the clotting parameter is recalcification time.

12.    A prepackaged diagnostic array, according to Claim 11, wherein the thrombotic index is obtained by dividing the value of the control sample in the first container by the value of the activated sample in the second container.

13.	A prepackaged diagnostic array, according to Claim 11, wherein a percentage shortening of clotting is obtained by

$$PSOC = \frac{R(s) - R(e) \times 100}{Rs}$$

wherein (s) is the value of the control sample in the first container and (e) is the value of the activated sample in the second container.

14.	A prepackaged diagnostic array, according to Claim 12, wherein the activating agent of the blood coagulation system is selected from the class consisting of endotoxins, collagens, platelet activating factors, carrageenans, thromboplastins, antigens and other activators of clotting or immunomodulars.

15.	A prepackaged diagnostic array, according to Claim 14, wherein the incubating temperature is approximately 37°C.

16.	A prepackaged diagnostic array, according to Claim 14, wherein the incubating time is approximately 2.0 hours.

17.	A prepackaged diagnostic array, according to Claim 14, wherein the vehicle in the first container is a saline solution.

18.	A prepackaged diagnostic array, according to Claim 14, wherein the activating agent in the second container is an endotoxin.